# EUROPÄISCHE PATENTSCHRIFT

(11) **EP 0 986 301 B1**
(45) Veröffentlichungstag und Bekanntmachung des Hinweises auf die Patenterteilung: **18.12.2002**
(21) Anmeldenummer: 98925629.2
(22) Anmeldetag: 22.05.1998
(51) Int. Cl.: A01N 37/18

(54) **INSEKTENVERTREIBUNGSMITTEL**
INSECT REPELLENT
INSECTIFUGE

(30) Priorität: 27.05.1997 DE 19722196
(43) Veröffentlichungstag der Anmeldung: 22.03.2000
(73) Patentinhaber: Engelhard Arzneimittel GmbH & Co. KG, 61138 Niederdorfelden (DE)
(72) Erfinder: RUNKEL, Frank, D-35418 Buseck (DE)
(74) Vertreter: Weller, Wolfgang, Dr.
(86) Internationale Anmeldenummer: EP9803008
(87) Internationale Veröffentlichungsnummer: WO98053683

(56) Entgegenhaltungen:
- EP-A- 0 251 464
- WO-A-97/49380
- GB-A- 1 575 387
- DATABASE WPI Section Ch, Week 9011 Derwent Publications Ltd., London, GB; Class B07, AN 90-079285 XP002077856 & JP 02 032190 A (OSAKA AEROSOL KOGYO KK) , 1. Februar 1990
- DATABASE WPI Section Ch, Week 8505 Derwent Publications Ltd., London, GB; Class A97, AN 85-027823 XP002077857 & JP 59 222402 A (OTSUKA KAGU KOGYO KK) , 14. Dezember 1984
- DATABASE WPI Section Ch, Week 9351 Derwent Publications Ltd., London, GB; Class A96, AN 93-410737 XP002077858 & JP 05 310508 A (KANEBO LTD) , 22. November 1993
- DATABASE WPI Section Ch, Week 9332 Derwent Publications Ltd., London, GB; Class B05, AN 93-252624 XP002077859 & JP 05 170603 A (KANEBO LTD) , 9. Juli 1993
- DATABASE WPI Section Ch, Week 8503 Derwent Publications Ltd., London, GB; Class B05, AN 85-017973 XP002077860 & SU 1 098 550 A (CHEMICALISATION) , 23. Juni 1984
- DATABASE CROPU STN-International STN-accession no. 97-87312, XP002077854 & ZA 9 506 460 A (PRETORIUS) 30. April 1997
- DATABASE WPI Section Ch, Week 9835 Derwent Publications Ltd., London, GB; Class A96, AN 98-411618 XP002077861 & RU 2 098 078 C (ALTAIKHIMPROM STOCK CO) , 10. Dezember 1997
- DATABASE WPI Section Ch, Week 8743 Derwent Publications Ltd., London, GB; Class A97, AN 87-302816 XP002077862 & JP 62 212302 A (FUKUBI CHEM IND CO LTD) , 18. September 1987
- DATABASE CROPU STN-International STN-accession no. 98-82905, H.QIU ET AL.: "Formulation of topical insect repellent N,N-diethyl-M-toluamide (deet): vehicle effects on deet in vitro skin permeation" XP002077855 & PHARM.RES., Bd. 14, Nr. 11, SUPPL., 1997, Seite S-313
- DATABASE WPI Section Ch, Week 9320 Derwent Publications Ltd., London, GB; Class B05, AN 93-162085 XP002077863 & JP 05 092905 A (SHISEIDO CO LTD) , 16. April 1993

## Beschreibung

Die Erfindung betrifft Insektenvertreibungsmittel, enthaltend N,N-Diethyl-m-toluamid (DEET) auf der Basis alkoholischer Lösungen als Wirkstoff (Repellent) und einen Hilfsstoff, der die Wirksamkeitsdauer von DEET, nach Auftragen auf der menschlichen Haut, verlängert.

Ein derartiges Insektenvertreibungsmittel ist aus der EP 0 090 288 B1 bekannt.

DEET (N,N-Diethyl-m-toluamid) ist ein bekanntes und bewährtes Insektenvertreibungsmittel, ein sogenanntes Repellent.

In den bekannten Insektenvertreibungsmitteln liegt das DEET als alkoholische Lösung vor. Wird eine solche alkoholische Lösung auf die Haut aufgetragen, beträgt die Wirksamkeitsdauer 5 Stunden.

Für eine wünschenswert lange Wirksamkeit im Bereich von 6 bis 8 Stunden nach Auftragen auf der menschlichen Haut wurden Hilfsstoffe gefunden, die die Wirksamkeitsdauer verlängern.

Bei der eingangs genannten EP 0 090 288 wurde herausgefunden, daß durch den Zusatz des Hilfsstoffes Polyethylenglykol 400 (PEG-400) eine deutlich verminderte Resorption und eine verbesserte Wirksamkeitsdauer des eigentlichen Wirkstoffes, also des Repellents DEET erreicht werden konnte. Der Hilfsstoff Polyethylenglykol 400 selbst besitzt keine Repellentwirkung. Durch den Zusatz des Hilfsstoffes PEG-400 zu alkoholischen Lösungen von DEET konnte die Wirksamkeitsdauer erhöht werden. So konnte die Wirksamkeitsdauer von 20 % DEET in Isopropanol durch den Zusatz von 20 % PEG-400 von 5,7 auf 7,4 Stunden erhöht werden. Aus der EP 0 090 288 ist es ferner bekannt geworden, als Lösungsmittel ein- oder mehrwertige Alkohole, bspw. auch Glycerin einzusetzen. Ein Ausführungsbeispiel beschreibt eine Formulierung aus 15 Teilen DEET als Wirkstoff (Repellent), 13 Teile PEG-400 als die Wirksamkeitsdauer verlängernder Hilfsstoff und einer Mischung aus 10 Teilen Glycerin, 8 Teilen Wasser, 53 Teilen Isopropanol als Lösungsmittel.

JP-A-2032190 beschreibt Aerosolformulierungen von Repellents wie DEET, 20-60% Ethanol, mit 0.1-10% einer verdampfungsverzögernden Substanz, z.B. Glycerin. Die Formulierungen bewirken eine verlängerte Wirkungsdauer.

JP-A-59222402 offenbart insektizide Zubereitungen, wobei flüchtige Wirkstoffe in Alkoholen formuliert sind. Dialkyltoluamide sind als Wirkstoff genannt und Glycerin ist als mögliche alkoholische Komponente genannt. Die Formulierungen enthalten vorzugsweise 5-60 Gew.-% Alkohol.

JP-A-5310508 offenbart auf Paraffin basierende Salben, die 3.0% DEET mit 10.0% Glycerin enthalten.

JP-A-5170603 offenbart auf Paraffin basierende Hautrepellents, die DEET und 10% Glycerol enthalten.

SU-A-1098550 offenbart Insektenrepellents, enthaltend DEET in Ethanol, mit 0.8-1 Gew.-% Glycerin.

EP-A-251464 offenbart Gelformulierungen von Pyrethroiden, wobei DEET, Glycerin und Alkohole in den Formulierungen enthalten sind. Nichtsdestoweniger ist die DEET nicht in alkoholischer Lösungen vorhanden, wie in den Formulierungen der vorliegende Ansprüche spezifiert ist.

ZA-A-9506460 offenbart Repellents, die einen Wirkstoff wie DEET in Zusammensetzung mit 1-50 Gew.-% einer Mischung aus Glycerin und Sorbitol enthalten.

Im praktischen Einsatz solcher Insektenvertreibungsmittel wurde nun festgestellt, daß eine Wirksamkeitsdauer im Bereich von 7 bis 8 Stunden nicht ausreichend ist, um eine über einen ganzen Tag gesehene dauerhafte Wirkung zu erzielen.

Insbesondere in den Sommermonaten, in denen die Insektenbelästigung auftritt, sind die Tage wesentlich länger als 8 Stunden, d.h. der Zeitraum, in dem eine Person im Laufe eines Tages Insekten ausgesetzt ist, ist wesentlich länger als 8 Stunden. Dies führt nun dazu, daß mit einer einmaligen Anwendung eines solchen Insektenvertreibungsmittels am Tag, bspw. am Morgen, nicht über den gesamten Tag eine Vertreibung der Insekten erzielt werden kann.

Der Einsatz von höheren Konzentrationen an DEET oder der Einsatz von höheren Konzentrationen an dem die Wirksamkeitsdauer verlängernden Hilfsstoff PEG-400 konnte keine wesentlichen Verbesserungen erzielen, bzw. die damit verbundenen Nachteile schließen solche Erhöhungen aus.

Nach der Monografie Diethyltoluamid (Bundesanzeiger Nr. 137 vom 23.07.1994) können bei Konzentrationen von mehr als 30 % als Nebenwirkungen Blasenbildung, Ulzerationen und Nekrosen auftreten.

Bei Polyethylenglykolen können gelegentlich Kontaktallergien auftreten, hierbei stehen die flüssigen Polyethylenglykole PEG-200 bis 400 im Vordergrund (A. Zesch Externa, Springer Verlag 1980).

Ferner zeigen Polyethylenglykole folgende unerwünschte Reaktionen, nämlich Peroxidbildung unter Sauerstoff- bzw. Lufteinwirkung, Reaktionen mit Stoffen, die phenolische OH-Gruppen enthalten, wie Kresole, Tannine, Resorcin und Salicylsäure, sie zeigen Verfärbungen mit Sulfonamiden, Anthrasol, Chrysarobin, Theophyllin.

Ferner führen PEGs zur Inaktivierung einiger Antibiotika und Konservierungsmittel, wie z.B. Penicilline und p-Hydroxybenzoesäureester. PEGs führen auch zur Auflösung von Kunststoffen wie Polyethylen, PVC und Zelluloseestern (Sucker et al.: Pharm. Technologie, 2. Auflage 1991).

Diese Reaktionen der PEG's führen somit zu einer chemischen Veränderung, die die Wirksamkeit und die Haltbarkeit des Insektenvertreibungsmittels beeinträchtigen können, aufgrund von Verfärbungen zu sichtbaren Verfärbungen der Hautbezirke führen, auf denen das Insektenvertreibungsmittel aufgetragen ist und möglicherweise andere auf der Haut aufgetragene oder in dieser enthaltene Mittel, wie bspw. Antibiotika oder Konservierungsmittel, beeinträchtigen.

In den Sommermonaten ist es üblich, Sonnenschutzmittel einzusetzen, die bspw. Konservierungsmittel enthalten. Reagiert nun das PEG mit einem Konservierungsmittel eines Sonnenschutzmittels, zeigt dieses Zersetzungserscheinungen, die nicht nur mit dem Verlust der Wirkung verbunden sind, sondern auch oft mit einer unangenehmen Geruchsbelästigung. Wird das Repellent auf einen Hautbezirk aufgetragen, der aufgrund einer Wunde mit Antibiotika bestrichen ist, können Beeinträchtigungen im Sinne einer Inaktivierung auftreten.

Vor diesem Hintergrund ist es Aufgabe der vorliegenden Erfindung, ein Insektenvertreibungsmittel der eingangs genannten Art mit einem Hilfsstoff zu versehen, der einerseits die Wirksamkeitsdauer von DEET beachtlich verlängert, andererseits von der menschlichen Haut ohne Wechselwirkungen vertragen wird und auch keinen unerwünschten Reaktionen eingeht.

Erfindungsgemäß wird die Aufgabe dadurch gelöst, daß der Hilfsstoff Glycerin ist.

Durch intensive Untersuchungen wurde nun in überraschenderweise festgestellt, daß Glycerin als Hilfsstoff einsetzbar ist, der die Wirksamkeitsdauer von DEET wirksam verlängert.

Es ist möglich, den Hilfsstoff PEG-400 durch Glycerin zu ersetzen, so daß Glycerin als einziger ausschließlicher, die Wirksamkeitsdauer von DEET verlängernder Hilfsstoff einsetzbar ist.

Diese Wirkung ist insbesondere deswegen als überraschend anzusehen, da, trotz des bekannten Einsatzes von Glycerin als Lösungsmittel, im Stand der Technik nach anderen, die Wirksamkeitsdauer von DEET verlängernden Hilfsstoffen gesucht wurde, was bei der eingangs genannten EP 0 090 288 zu dem Hilfsstoff PEG-400 geführt hat. Es ist daher als überraschend anzusehen, daß Glycerin als einziger, somit ausschließlicher Hilfsstoff zur Verlängerung der Wirksamkeitsdauer von DEET-Formulierungen auf alkoholischer Basis eingesetzt werden kann.

Glycerin ist ein physiologischer Stoff, der im Stoffwechsel des menschlichen Organismus auftritt. In Dermatika wird Glycerin bei angegriffener, spröder ausgetrockneter Haut als hautpflegender Zusatz eingesetzt. Glycerin zeigt auch bei hochsensiblen Allergikern keinerlei Kontaktallergien, so daß ein Einsatz auch bei diesen empfindlichen Personen möglich ist. Es ist zu bedenken, daß solche Insektenvertreibungsmittel meist in den Urlaubsmonaten eingesetzt werden, in denen man in einer fremden Umgebung, einer hohen Belastung an Mücken, und auch meist höheren Temperaturen mit intensiver Sonneneinstrahlung ausgesetzt ist. Personen mit empfindlicher Haut reagieren dann auf zusätzlich auf die Haut aufgetragene Mittel, wie bspw. ein Insektenvertreibungsmittel, mit allergischen Reaktionen.

Intensive Untersuchungen haben gezeigt, daß mit Glycerin als Hilfsstoff eines Insektenvertreibungsmittels solche Reaktionen nicht auftreten. Auch die eingangs erwähnten Reaktionen, nämlich Peroxidbildung bei Sauer- und Lufteinwirkung, Reaktionen mit Stoffen, die phenolische OH-Gruppen enthalten, Verfärbung mit Sulfonamiden, Inaktivierung von Antibiotika oder Konservierungsmitteln, treten bei Glycerin als Hilfsstoff nicht auf.

Somit wird nicht nur ein sehr hautverträglicher Hilfsstoff zur Verfügung gestellt, sondern es besteht die Möglichkeit, diesen in relativ hohen Konzentrationen einzusetzen, was zu einer weiteren Steigerung der Wirksamkeitsdauer führt, ohne durch die Erhöhung der Konzentration unerwünschte Nebenwirkungen auszulösen.

Ein als noch weit überraschenderer Effekt des Ersatzes von PEG-400 durch Glycerin ist darin zu sehen, daß, bei vergleichbaren Mengen an Wirkstoff DEET und vergleichbaren Mengen an Hilfsstoff, die Wirksamkeitsdauer eines Insektenvertreibungsmittels mit dem Hilfsstoff Glycerin bis auf 13 Stunden erhöht werden kann. Es erfolgt also eine Erhöhung um 7 Stunden gegenüber der üblichen Wirksamkeitsdauer ohne Hilfsstoffe, und um weitere 5 Stunden gegenüber dem Hilfsstoff PEG-400.

Dies führt im Gesamtergebnis zu einer wesentlichen Erhöhung der Wirksamkeitsdauer bei gleichzeitiger Verringerung bzw. Nichtauftreten von Nebenwirkungen, insbesondere allergischen Reaktionen und Reaktionen mit Umweltstoffen oder in der Haut enthaltenen natürlichen oder anderen aufgetragenen Stoffen.

In einer bevorzugten Ausgestaltung der Erfindung beträgt der Anteil an Glycerin mehr als 10 Gew.-% bezogen auf das Gesamtgewicht des Insektenvertreibungsmittels.

Diese Maßnahme hat den Vorteil, daß mit Gehalten von mehr als 10 Gew.-% Glycerin schon eine beachtliche Verlängerung der Wirksamkeitsdauer erzielt werden kann.

Der Anteil an Glycerin kann bis zu 60 Gew.-% betragen, er beträgt vorzugsweise 20 bis 50 Gew.-%.

In diesem vorzugsweisen Bereich wird eine optimale Verlängerung der Wirksamkeitsdauer erzielt, die Wirksamkeitsdauer beträgt bis zu 13 Stunden, d.h. es kann auch bei einem sehr langen und sonnigen Sommertag mit einer einzigen Anwendung am Morgen eine durchgehende insektenvertreibende Wirkung erzielt werden.

In einer weiteren Ausgestaltung der Erfindung beträgt der Anteil an DEET 15 bis 25 Gew.-%.

Diese Maßnahme hat den Vorteil, daß mit einer Menge an Wirkstoff (Repellent) gearbeitet werden kann, die einerseits ausreichend ist, um eine optimale Wirksamkeit über die lange Dauer zu erzielen, gleichzeitig eine solche geringe Menge darstellt, daß die in der Monografie beschriebenen Nebenwirkungen wie Blasenbildung, Ulzeration und Nekrosen ausgeschlossen sind.

In einer weiteren Ausgestaltung der Erfindung sind im Insektenvertreibungsmittel weitere Repellentien enthalten.

Diese an sich bekannte Maßnahme hat den Vorteil, daß in besonderen Fällen, in denen die Wirksamkeit von DEET aurgrund örtlicher oder sonstiger Gegebenheiten unterstützt werden muß, weitere Repellentien herangezogen werden können.

In einer weiteren Ausgestaltung der Erfindung sind Zusatzstoffe, ausgewählt aus der Gruppe bestehend aus Parfümölen, Riechstoffen, hautpflegenden Stoffen, Wasser, Emulgatoren, Treibstoffen enthalten.

Diese Maßnahme hat den Vorteil, daß aufgrund der Zusatzstoffe Formulierungen bereitgestellt werden können, die angenehm auf der Haut sind, oder besondere Galeniken angeboten werden können, bspw. als Hautspray, als Lotion, als Creme oder als Stift.

Die erfindungsgemäße Verwendung von Glycerin als die Wirksamkeitsdauer verlängernder Hilfsstoff in einem Insektenvertreibungsmittel führt somit zu einer Verlängerung der Wirksamkeitsdauer bei relativ geringen Mengen an eigentlichem Wirkstoff (Repellent), so daß ein wirksames und zugleich verträgliches Insektenvertreibungsmittel geschaffen ist.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine vergleichende Darstellung der Wirkungsdauer in Form eines Balkendiagramms einer 20%-igen DEET-Formulierung in Abhängigkeit der Glycerinkonzentration, und
- Fig. 2: eine vergleichende Darstellung der Fernhaltewirkung von Insektenvertreibungsmittel mit PEG-400 und mit Glycerin als Wirkstoff in Form eines perspektivischen Balkendiagramms.

### PRÜFMETHODE

Die Prüfung der Wirksamkeitsdauer der nachfolgend beschriebenen Formulierungen erfolgte nach einem bewährten Testverfahren des Schweizerischen Tropeninstitutes. Als Versuchstiere dienten etwa 300 bis 400 Gelbfiebermücken (*Aedes, aegypti)* aus der Laborzucht, und zwar ausschließlich Weibchen. Die Prüfungen erfolgten in einem Zuchtkäfig von 40 x 40 x 40 cm. Um sicherzugehen, daß alle Mückenweibchen während der Testphase hungrig sind, wurden ihnen am Abend vor dem Testtag das Zuckerwasser entzogen. Testbeginn ist jeweils zwischen 8 Uhr und 10 Uhr vormittags.

Am Unterarm einer Versuchsperson werden ca. 250 cm² mit der zu untersuchenden Probe behandelt. Dazu wurde eine Menge von 2 ml der entsprechenden Probe gleichmäßig verteilt. Die behandelte Unterarmfläche wurde an beiden Enden mit einem mükkendichten Klebeband und einem kurzen Plastikschlauch abgedichtet. Die unbehandelte Hand wurde mit dicken Handschuhen überzogen und diente gleichzeitig als Kontrolle für die Stechaktivität der Mücken.

Es wurde ferner Sorge dafür getragen, daß die mit der Probe behandelten Flächen während der Versuchszeit unberührt blieben.

Für den eigentlichen Test wurde der Unterarm und entsprechend die Haut stündlich in den Mückenkäfig gehalten und während einer Testdauer von 10 Minuten die Zahl der Stechmücken notiert, die
a) durch den Handschuh zu stechen versuchten (positive Kontrolle),
b) die behandelte Fläche näher als 3 cm anflogen (zu Beginn, Testmitte, und am Ende),
c) auf der behandelten Fläche länger als 2 Sekunden sitzen blieben, und
d) in die behandelte Fläche einstachen und Blut saugten.

### Auswertung und Interpretation

Die Wirksamkeit einer Probe ergibt sich vor allem aus dem Verhältnis der auf dem Handschuh absitzenden stechlustigen Mücken zu den übrigen Werten.

Die Zahl der absitzenden Mücken ist deswegen von Bedeutung, da schon die in der Nähe der Person umherschwirrenden oder gar absitzenden Mücken eine gewisse Belästigung darstellen.

Die Zahl der einstechenden Mücken ist für die Dauer der Wirksamkeit und damit in den Tropen indirekt für die Infektionsgefahr der entscheidende Faktor.

### FORMULIERUNGEN

| Formulierung ohne Glycerin | | |
|---|---|---|
| 100 ml enthalten folgende Inhaltsstoffe: | | |

| | Einwaagen in [g] | |
|---|---|---|
| N,N-Diethyl-m-toluamid USP XXII | 19,000 | (Repellent) |
| Ethanol 96 % DAB 10 | 38,000 | (Lösungsmittel) |
| Isopropylmyristat EP | 28,230 | (Zusatzstoff) |
| Parfümöl Bianca | 0,240 | (Zusatzstoff) |
| | 85,470 | |
| Dichte: 0,8547 g/ml | | |

Isopropylmyristat stellt einen Zusatzstoff dar, der dafür sorgt, daß beim Auftragen des Insektenvertreibungsmittels ein angenehmes Gefühl auf der Haut entsteht.

| Formulierung mit 10 % Glycerin | | |
|---|---|---|
| 100 ml enthalten folgende Inhaltsstoffe: | | |

| | Einwaagen in [g] | |
|---|---|---|
| N,N-Diethyl-m-toluamid USP XXII | 19,000 | (Repellent) |
| Ethanol 96 % DAB 10 | 38,000 | (Lösungsmittel) |
| Glycerin 85 % EP (pflanzlich) | 11,000 | (Hilfsstoff) |
| Isopropylmyristat EP | 21,100 | (Zusatzstoff) |
| Parfümöl Bianca | 0,240 | (Zusatzstoff) |
| | 89,340 | |
| Dichte: 0,8934 g/ml | | |

| Formulierung mit 40 % Glycerin | | |
|---|---|---|
| 100 ml enthalten folgende Inhaltsstoffe: | | |

| | Einwaagen in [g] | |
|---|---|---|
| N,N-Diethyl-m-toluamid USP XXII | 19,000 | (Repellent) |
| Ethanol 96 % DAB 10 | 38,000 | (Lösungsmittel) |
| Glycerin 85 % EP (pflanzlich) | 41,640 | (Hilfsstoff) |
| Parfümöl Bianca | 0,240 | (Zusatzstoff) |
| | 98,880 | |
| Dichte: 0,9888 g/ml | | |

Die drei vorgenannten Formulierungen wurden wie zuvor beschrieben auf die Haut appliziert und der beschriebene Test wurde durchgeführt. Das Ende der Wirkungsdauer wird angenommen, wenn während einer Testspanne in die behandelte Fläche mindestens zwei Mücken einstechen und Blut saugen.

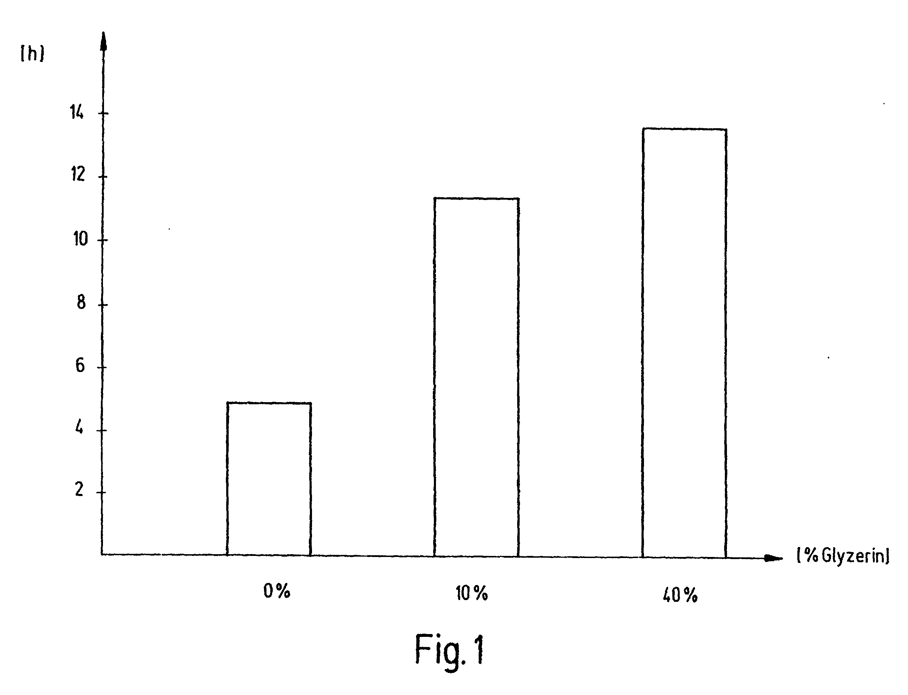

In Fig. 1 sind die Testergebnisse der drei Formulierungen, nämlich Formulierung ohne Glycerin, Formulierung mit 10 % Glycerin und Formulierung mit 40 % Glycerin dargestellt. Auf der Ordinate ist die Wirksamkeitsdauer in Stunden aufgetragen.

Daraus ist ersichtlich, daß die Wirkungsdauer schon bei einem Gehalt von etwa 10 % Glycerin auf 11 Stunden gegenüber einer Formulierung ohne Glycerin (5 Stunden) erhöht wird. Bei einer Formulierung mit 40 % Glycerin konnte die Wirkungsdauer auf 13 Stunden erhöht werden.

In einer weiteren Testreihe wurde ein Insektenvertreibungsmittel mit dem Hilfsstoff PEG-400 mit einer erfindungsgemäßen Formulierung mit dem Hilfsstoff Glycerin verglichen.

Die Formulierung aus dem Stand der Technik ist in Deutschland unter der als Marke geschützten Bezeichnung AUTAN erhältlich und enthält 20 g DEET als Wirkstoff. Der Gehalt an PEG-400 liegt im Bereich zwischen 20 und 40 %.

Diesem bekannten Insektenvertreibungsmittel wurde eine erfindungsgemäße Formulierung mit 20 % DEET als Wirkstoff (Repellent) und 35 % Glycerin als Hilfsstoff gegenübergestellt.

Es wurden mit beiden Formulierungen der zuvor erwähnte Test durchgeführt.

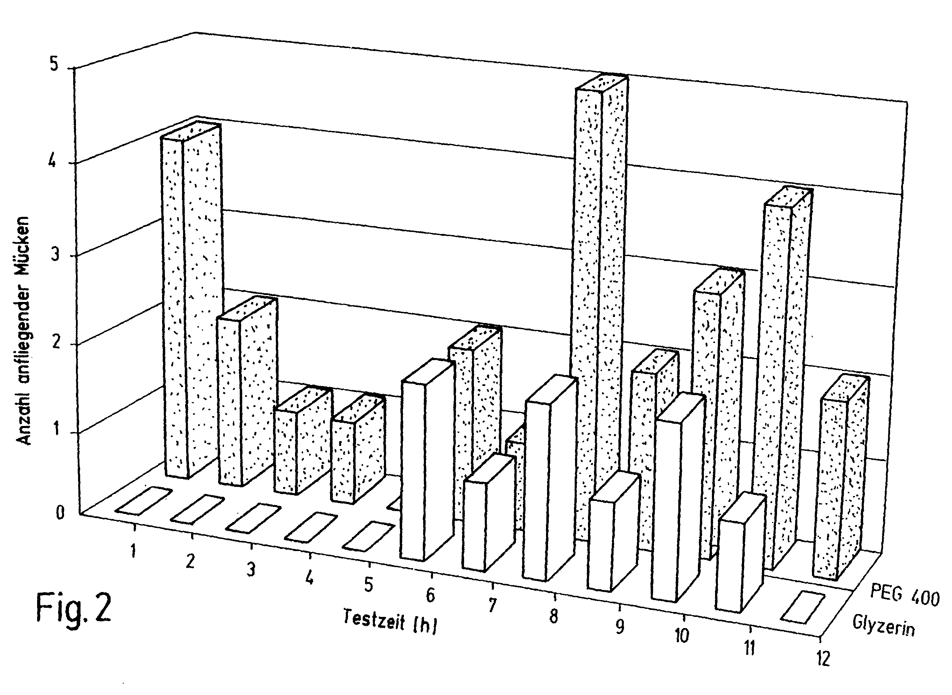

In Fig. 2 ist die Fernhaltewirkung der beiden gegenübergestellten Insektenvertreibungsmittel dargestellt, und zwar angegeben in der Anzahl der die behandelte Fläche näher als 3 cm anfliegenden Stechmücken in Abhängigkeit von der Testzeit.

Daraus ist zu erkennen, daß mit der Glycerin-Formulierung nicht nur in den ersten Stunden der Testzeit, sondern auch 8 bis 12 Stunden nach Auftragen eine beachtliche Fernhaltewirkung erzielt werden kann, was besonders beeindruckend die Langzeitwirkung demonstriert.

## Patentansprüche

1. Insektenvertreibungsmittel, enthaltend N,N-Diethyl-mtoluamid (DEET) auf der Basis alkoholischer Lösungen als Wirkstoff (Repellent) und einen Hilfsstoff, der die Wirksamkeitsdauer von DEET, nach Auftragen auf der menschlichen Haut, verlängert, **dadurch gekennzeichnet, daß** es als einzigen, die Wirksamkeitsdauer verlängernden Hilfsstoff Glycerin enthält und daß der Anteil an Glycerin mehr als 10 Gew.-% bezogen auf das Gesamtgewicht des Insektenvertreibungsmittels beträgt.

2. Insektenvertreibungsmittel nach Anspruch 1, **dadurch gekennzeichnet, daß** der Anteil an Glycerin bis 60 Gew.-%, vvrzugsweise 20 bis 50 Gew.-% beträgt.

3. Insektenvertreibungsmittel nach Anspruch 1 oder 2, **dadurch gekennzeichnet, daß** der Anteil an DEET 15 bis 25 Gew.-% beträgt.

4. Insektenvertreibungsmittel nach einem der Ansprüche 1 bis 3, **dadurch gekennzeichnet, daß** weitere Repellentien enthalten sind.

5. Insektenvertreibungsmittel nach einem der Ansprüche 1 bis 4, **dadurch gekennzeichnet, daß** Zusatzstoffe, ausgewählt aus der Gruppe bestehend aus Parfümölen, Riechstoffen, hautpflegenden Stoffen, Wasser, Emulgatoren, Treibstoffen, enthalten sind.

6. Verwendung von Glycerin als einzigem die Wirksamkeitsdauer verlängernden Hilfsstoff in einem Insektenvertreibungsmittel, das N-N-Diethyl-m-toluamid (DEET) auf der Basis alkoholischer Lösung als Wirkstoff (Repellent) enthält, wobei der Anteil an Glycerin mehr als 10 Gew.-% bezogen auf das Gesamtgewicht des Insektenvertreibungsmittels beträgt.

7. Verwendung nach Anspruch 6, **dadurch gekennzeichnet, daß** der Anteil an Glycerin bis 60 Gew.-%, vorzugsweise 20 bis 50 Gew.-% beträgt.

8. Verwendung nach Anspruch 6 oder 7, **dadurch gekennzeichnet, daß** der Anteil an DEET 15 bis 25 Gew.-% beträgt.

9. Verwendung nach einem der Ansprüche 6 bis 8, **dadurch gekennzeichnet, daß** weitere Repellentien im Insektenvertreibungsmittel enthalten sind.

10. Verwendung nach einem der Ansprüche 6 bis 9, **dadurch gekennzeichnet, daß** Zusatzstoffe, ausgewählt aus der Gruppe bestehend aus Parfümölen, Riechstoffen, hautpflegenden Stoffen, Wasser, Emulgatoren, Treibstoffen im Insektenvertreibungsmittel enthalten sind.

## Claims

1. Insect-repelling agent containing N,N-diethyl-m-toluamide (DEET) based on alcohol solutions as the active ingredient (repellent), and an adjuvant that extends the period of effectiveness of DEET following application to human skin, **characterized in that** the sole adjuvant extending the period of effectiveness contained therein is glycerol and the proportion of glycerol is more than 10 wt% in terms of the total weight of the insect-repelling agent.

2. Insect-repelling agent of Claim 1, **characterized in that** the proportion of glycerol is up to 60 wt%, preferably 20 to 50 wt%.

3. Insect-repelling agent of Claims 1 or 2, **characterized in that** the proportion of DEET is 15 to 25 wt%.

4. Insect-repelling agent of anyone of Claims 1 through 3, **characterized in that** further repellents are contained therein.

5. Insect-repelling agent of anyone of Claims 1 through 4, **characterized in that** additives selected from the group consisting of perfume oils, fragrances, skin-care substances, water, emulsifiers, and propellants are contained therein.

6. Use of glycerol as the sole adjuvant that extends the period of effectiveness in an insect-repelling agent that contains N,N-diethyl-m-toluamide (DEET) based on an alcohol solution as the active ingredient (repellent), the proportion of glycerol being more than 10 wt% in terms of the total weight of the insect-repelling agent.

7. Use of Claim 6, **characterized in that** the proportion of glycerol is up to 60 wt%, preferably 20 to 50 wt%.

8. Use of Claims 6 or 7, **characterized in that** the proportion of DEET is 15 to 25 wt%.

9. Use of anyone of Claims 6 through 8, **characterized in that** further repellents are contained in the insect-repelling agent.

10. Use of anyone of Claims 6 through 9, **characterized in that** additives selected from the group consisting of perfume oils, fragrances, skin-care substances, water, emulsifiers, and propellants are contained in the insect-repelling agent.

## Revendications

1. Insectifuge contenant du N,N-diéthyl-m-toluamide (DEET) à base de solutions alcooliques sous forme de substance active (répulsif) et une substance auxiliaire, qui prolonge la durée d'efficacité du DEET, après application sur la peau humaine, **caractérisé en ce qu'**il contient de la glycérine en tant que seule substance auxiliaire prolongeant la durée d'efficacité et **en ce que** la proportion de glycérine s'élève à plus de 10 % en poids par rapport au poids total de l'insectifuge.

2. Insectifuge selon la revendication 1, **caractérisé en ce que** la proportion de glycérine s'élève jusqu'à 60 % en poids, de préférence de 20 à 50 % en poids.

3. Insectifuge selon la revendication 1 ou 2, **caractérisé en ce que** la proportion de DEET s'élève entre 15 et 25 % en poids.

4. Insectifuge selon l'une quelconque des revendications 1 à 3, **caractérisé en ce qu'**il contient d'autres répulsifs.

5. Insectifuge selon l'une quelconque des revendications 1 à 4, **caractérisé en ce qu'**il contient des additifs, choisis dans le groupe comprenant des huiles parfumées, des parfums, des substances nourrissant la peau, de l'eau, des émulsifiants, des essences.

6. Utilisation de glycérine en tant que seule substance auxiliaire prolongeant la durée d'efficacité d'un insectifuge, qui contient du N,N-diéthyl-m-toluamide (DEET) à base d'une solution alcoolique sous forme de substance active (répulsif), moyennant quoi la proportion de glycérine s'élève à plus de 10 % en poids par rapport au poids total de l'insectifuge.

7. Utilisation selon la revendication 6, **caractérisée en ce que** la proportion de glycérine s'élève jusqu'à 60 % en poids, de préférence entre 20 et 50 % en poids.

8. Utilisation selon la revendication 6 ou 7, **caractérisée en ce que** la proportion de DEET s'élève de 15 à 25 % en poids.

9. Utilisation selon l'une quelconque des revendications 6 à 8, **caractérisée en ce que** d'autres répulsifs sont contenus dans l'insectifuge.

10. Utilisation selon l'une quelconque des revendications 6 à 9, **caractérisée en ce que** des additifs choisis dans le groupe comprenant des huiles parfumées, des parfums, des substances pour le soin de la peau, de l'eau, des émulsifiants, des essences, sont contenus dans l'insectifuge.
